(19) Europäisches Patentamt
European Patent Office
Office européen des brevets

(11) Veröffentlichungsnummer : **0 473 550 A1**

(12) **EUROPÄISCHE PATENTANMELDUNG**

(21) Anmeldenummer : **91810667.5**

(22) Anmeldetag : **21.08.91**

(51) Int. Cl.[5] : **C07D 471/16,** A61K 31/47, // (C07D471/16, 221:00, 221:00, 209:00)

(30) Priorität : **27.08.90 DE 4027018**
**27.08.90 DE 4027015**

(43) Veröffentlichungstag der Anmeldung :
**04.03.92 Patentblatt 92/10**

(84) Benannte Vertragsstaaten :
**AT BE CH DE DK ES FR GB GR IT LI LU NL SE**

(71) Anmelder : **SANDOZ LTD.**
**Lichtstrasse 35**
**CH-4002 Basel (CH)**
(84) **BE CH DK ES FR GB GR IT LI LU NL SE**

(71) Anmelder : **SANDOZ-PATENT-GMBH**
**Humboldtstrasse 3**
**W-7850 Lörrach (DE)**
(84) **DE**

(71) Anmelder : **SANDOZ-ERFINDUNGEN**
**Verwaltungsgesellschaft m.b.H.**
**Brunner Strasse 59**
**A-1235 Wien (AT)**
(84) **AT**

(72) Erfinder : **Nozulak, Joachim**
**In der Ziegelei 1**
**W-7843 Heitersheim (DE)**

(54) **Indolonaphthyridine.**

(57) Indolonaphthyridine der Formel I,

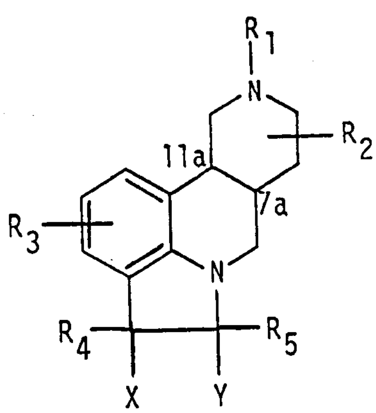

I

worin $R_1$, $R_2$, $R_3$, $R_4$, $R_5$, X und Y wie in der Beschreibung definiert sind, ihre Herstellung und Anwendung als Therapeutika.

EP 0 473 550 A1

Gegenstand der vorliegenden Anmeldung sind neue 7a,8,9,10,11,11a-Hexahydro- und 4,5,7a,8,9,10,11,11a-Octahydro-7H-indolo[1,7-bc]-[2,6]naphthyridine, ihre Herstellung und Anwendung bei der therapeutischen Behandlung.

Diese im folgenden als neue Verbindungen bezeichneten Substanzen entsprechen insbesondere der Formel I,

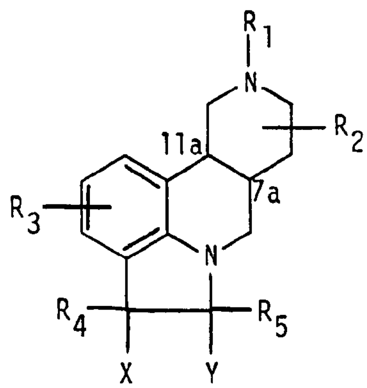

I

worin

$R_1$ Wasserstoff, Alkyl, Alkylcarbonylalkyl, Arylcarbonylalkyl, Aralkyl oder gegebenenfalls durch Alkyl oder Aryl mono- oder disubstituiertes Carbamoylalkyl bedeutet,

$R_2$ für eine der unter $R_1$ angegebenen Bedeutungen steht und zusätzlich Trifluormethyl, Alkoxy oder Alkylthio bedeuten kann,

$R_3$, $R_4$ und $R_5$ unabhängig voneinander je Wasserstoff, Halogen, Alkyl, Alkoxy, Alkylthio oder Trifluormethyl bedeuten und

X und Y je für Wasserstoff stehen oder zusammen eine einfache Bindung bilden,

in Form der freien Basen oder von Säureadditionssalzen.

Die neuen Verbindungen können je nach den vorhandenen Substituenten zwei (in Stellung 7a und 11a) oder mehr asymmetrische Kohlenstoffatome aufweisen. Die Erfindung beinhaltet alle daraus entstandenen Stereomere sowie ihre Gemische, beispielsweise die Racemate der Enantiomere.

In Stellung 7a und 11a können die neuen Verbindungen sowohl ciswie auch trans-verknüpft sein.

Die in den neuen Verbindungen enthaltenen Alkyl-, Alkoxy- und Alkylthiogruppen sowie die Alkylteile in den Alkylcarbonylalkyl, Arylcarbonylalkyl, Aralkyl und gegebenenfalls substituierten Carbamoylalkyl-Gruppen enthalten 1 bis 4 Kohlenstoffatome.

Ein Arylrest in einer Arylcarbonylalkyl-, Aralkyl- oder Arylcarbamoylalkylgruppe steht für ein gegebenenfalls durch oben definiertes Alkyl, Alkoxy oder Alkylthio mono-, di- oder trisubstituierter fünf- oder sechsgliedriger, gesättigter, ungesättigter oder aromatischer carbocyclischer Ring, wobei ein oder zwei Kohlenstoffatome durch Stickstoff ersetzt sein können.

Halogen steht für Fluor, Chlor, Brom oder Jod.

Die in den neuen Verbindungen enthaltenen, vorstehend definierten Alkyl-, Alkoxy- und Alkylthiogruppen enthalten vorzugsweise 1 oder 2 Kohlenstoffatome und stellen insbesondere Methyl, Methoxy und Methylthio dar.

Ein vorstehend definiertes Halogen steht vorzugsweise für Fluor oder Chlor.

$R_1$ steht vorzugsweise für Alkyl, insbesondere Methyl. $R_2$, $R_3$, $R_4$ und $R_5$ stehen vorzugsweise für Wasserstoff. Bevorzugt stehen X und Y je für Wasserstoff.

Die Erfindung umfasst z.B. eine Gruppe von Verbindungen der Formel I, worin $R_1$ Alkyl mit 1 bis 4 Kohlenstoffatomen bedeutet und $R_2$, $R_3$, $R_4$, $R_5$, X und Y je Wasserstoff bedeuten, in Form der freien Basen oder von Säureadditionssalzen.

Ferner umfasst die Erfindung eine Gruppe von Verbindungen der Formel I, worin $R_1$ Alkyl mit 1 bis 4 Kohlenstoffatomen bedeutet, $R_2$, $R_3$, $R_4$ und $R_5$ je Wasserstoff bedeuten und X und Y zusammen eine einfache Bindung bilden, in Form der freien Basen oder von Säureadditionssalzen.

Die bevorzugte Verbindung ist das (+)-cis-4,5,7a,8,9,10,11,11a-Octahydro-7H-10-methyl-indolo[1,7-bc][2,6]naphthyridin in Form der freien Base oder eines Säureadditionssalzes.

Erfindungsgemäss gelangt man zu den neuen Verbindungen und ihren Säureadditionssalzen, indem man

a) zur Herstellung der Verbindungen der Formel Ia,

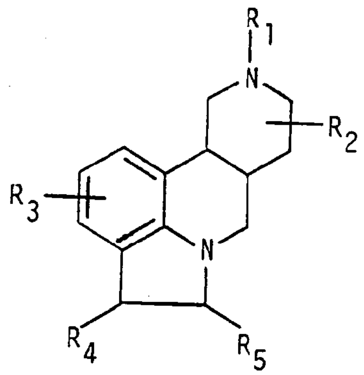

Ia

worin $R_1$, $R_2$, $R_3$, $R_4$ und $R_5$ obige Bedeutung besitzen, in Verbindungen der Formel II,

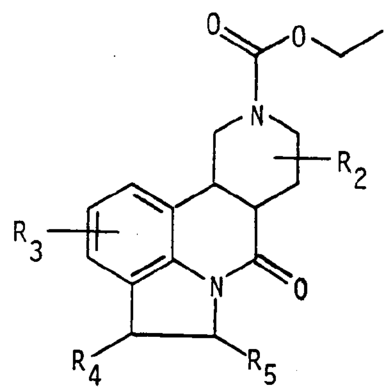

II

worin $R_2$, $R_3$, $R_4$ und $R_5$ obige Bedeutung besitzen, die 10-Ethoxycarbonylgruppe unter Reduktion der 7-Oxogruppe durch die Gruppe $R_1$ ersetzt, oder
b) zur Herstellung der Verbindungen der Formel Ib,

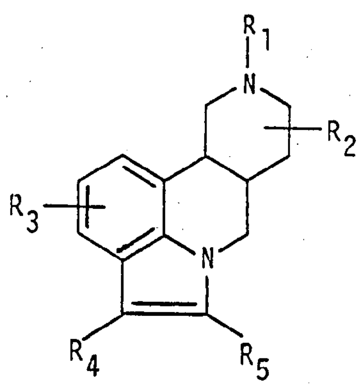

Ib

worin $R_1$, $R_2$, $R_3$, $R_4$ und $R_5$ obige Bedeutung besitzen, Verbindungen der Formel Ia oxidiert,
und die erhaltenen Verbindungen der Formel I in Form der freien Basen oder in Säureadditionssalzform gewinnt.

Die Reaktion gemäss Verfahren a) kann nach an sich bekannten Methoden durchgeführt werden.

Zur Einführung einer Methylgruppe kann die Ethoxycarbonylgruppe z.B. mittels Aluminiumhydrid oder komplexen Alumlniumhydriden wie Lithiumaluminiumhydrid reduziert werden.

Zur Einführung einer höheren Alkylgruppe oder einer substituierten Alkylgruppe kann zuerst die Ethoxygruppe abgespaltet und dann das erhaltene Amin alkyliert werden.

Die Oxidation der Verbindungen der Formel Ia gemäss Verfahren b) kann nach an sich bekannten Methoden durchgeführt werden, zum Beispiel mittels Manganoxid. Es können auch $O_2$/Kobaltsalze in Methanol, Palladiumdichlorid in Triethylamin oder Benzolseleninsäureanhydrid verwendet werden.

Die Aufarbeitung der erhaltenen Reaktionsgemische und die Reinigung der so gewonnenen Verbindungen der Formel I kann nach an sich bekannten Methoden durchgeführt werden.

Aus den freien Basen lassen sich in bekannter Weise Säureadditionssalze herstellen und umgekehrt.

Die erfindungsgemässen Verfahren können mit Ausgangsprodukten in Form der individuellen, optisch aktiven Isomeren oder deren Isomergemische, besonders deren Racemate, erfolgen und führen dann zu den entsprechenden Endprodukten.

Die Racemate können in die individuellen, optisch aktiven Komponenten gespalten werden, wobei bekannte Methoden zur Anwendung gelangen, z.B. via vorübergehende Säureadditionssalzbildung mit optisch aktiven Säuren, z.B. (+)-[bzw. (-)]-Di-O,O′-p-toluoyl-D-(-)-[bzw. L-(+)]-weinsäure, und fraktionierter Kristallisation der diastereoisomeren Säureadditionssalze.

Die Ausgangsverbindungen der Formel II können ausgehend von Isonicotinsäurechlorid (bekannt aus Beilstein 4 Vol. 22/1, S. 526) und Indolin (bekannt aus Beilstein 4, Vol. 20/4, S. 2896) oder von nach an sich bekannten Methoden herstellbaren Derivaten davon, nach dem folgenden Reaktionsschema, z.B. wie im Beispiel 1 unter a) bis e) beschrieben, hergestellt werden:

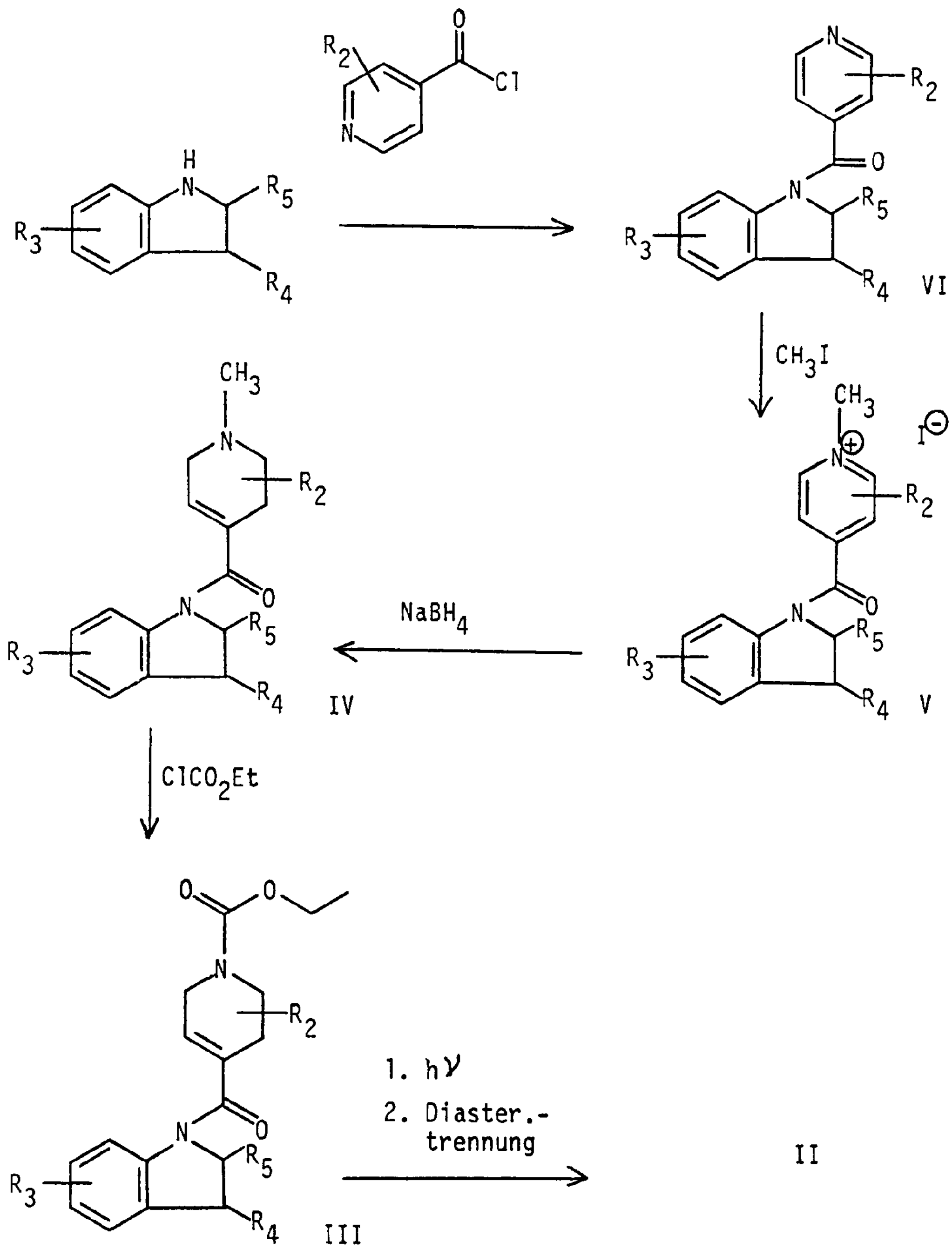


Die 7a,8,9,10,11,11a-Hexahydro- und 4,5,7a,8,9,10,11,11a-Octahydro-7H-indolo[1,7-bc][2,6]naphthyridine und ihre physiologisch verträglichen Säureadditionssalze, im folgenden als erfindungsgemässe Verbindungen bezeichnet, weisen im Tierversuch interessante pharmakologische Eigenschaften auf und können daher als Heilmittel verwendet werden.

Insbesondere bewirken die erfindungsgemässen Verbindungen eine antagonistische Wirkung an den zentralen 5HT-1C Rezeptoren.

Die erfindungsgemässen Verbindungen weisen eine starke Bindungsaffinität zu zentralen 5HT-1C Rezeptoren auf, die nach der Methode von D. Hoyer et al., Eur. J. Pharm., 118, 13 - 23 (1985) gemessen wurde. Die

Verbindung des nachfolgenden Beispiels 5 hat in diesem Versuch einen pKD-Wert von 7,8.

Ferner antagonisieren die erfindungsgemässen Verbindungen die in Ratten durch Verabreichung von m-Chlorophenyl-piperazin (mCPP) nach der Methode von G. A. Kennett und G. Curzon, Br. J. Pharmacol., 94, 137 - 147 (1988) hervorgerufene Hypolokomotion. Diese Aktivität wird bei Dosen von ca. 0,5 bis 30 mg/kg p.o. festgestellt. Die Verbindung des nachfolgenden Belspiels 5 hat in diesem Versuch einen $ED_{50}$-Wert von 3 mg/kg p.o.

Aufgrund dieser Wirkungen können die erfindungsgemässen Verbindungen für die prophylaktische Behandlung der Migräne oder für die Behandlung von Störungen wie z.B. Angst, Depression, Schizophrenie, Autismus, Panikattacken, Zwangskrankheiten, Priapismus, Bulimie und Zuständen mit erhöhtem intracranialem Druck eingesetzt werden.

Eine geeignete Tagesdosis liegt im Bereich von etwa 0,5 bis 300 mg der Substanz, geeignete Dosierungsformen für z.B. orale Anwendungen enthalten im allgemeinen ungefähr 0,10 bis 150 mg wirksame Substanz neben festen oder flüssigen Trägersubstanzen oder Verdünnungsmitteln.

Als Heilmittel können die erfindungsgemässen Verbindungen allein oder in geeigneter Arzneiform mit pharmakologisch indifferenten Stoffen verabreicht werden.

Gegenstand der Erfindung sind auch pharmazeutische Zusammensetzungen, die die erfindungsgemässen Verbindungen als Wirkstoffe enthalten. Für ihre Herstellung können die in der Pharmazie gebräuchlichen Hilfs- und Trägerstoffe verwendet werden. Geeignete galenische Formen sind z.B. Tabletten, Kapseln und Tropflösungen.

Die nachfolgenden Beispiele erläutern die Erfindung. Temperaturen erfolgen in Celsiusgraden und sind unkorrigiert.

**BEISPIEL 1: cis-4,5,7a,8,9,10,11,11a-Octahydro-7H-10-methyl-indolo[1,7-bc][2,6]naphthyridin**

2,03 g (53,4 mmol) Lithiumaluminumhydrid (es kann auch Aluminiumhydrid verwendet werden) werden bei 0 ° in absolutem Tetrahydrofuran vorgelegt. Anschliessend tropft man eine Loesung von 1,61 g (5,36 mmol) cis-4,5,7a,8,9,10,11,11a-Octahydro-7-oxo-7H-indolo-[1,7-bc][2,6]naphthyridin-10-carbaminsaeureethylester in absolutem Tetrahydrofuran ein. Die Reaktionsmischung wird drei Stunden zum Rueckfluss erhitzt und anschliessend 12 Stunden bei Raumtemperatur stehen gelassen. Danach kuehlt man auf - 20 ° und tropft Wasser in die Reaktionsloesung. Das Produkt wird mit Toluol extrahiert. Die Toluolphase wird getrocknet und eingedampft. Man erhaelt als gelbes Oel cis-4,5,7a,8,9,10,11,11a-Octahydro-7H-10-methyl-indolo[1,7-bc][2,6] naphthyridin. Das Rohprodukt wird in Aceton geloest und mit der equivalenten Menge Malonsaeure versetzt. Man erhaelt nach Kristallisation aus Methanol/Aceton das cis-4,5,7a,8,9,10,11,11a-Octahydro-7H-10-methyl-indolo[1,7-bc][2,6]naphthyridin-malonat mit dem Schmelzpunkt 158 - 159 °.

Das Ausgangsmaterial kann wie folgt hergestellt werden:

**a) 2,3-Dihydro-1-(4-pyridinylcarbonyl)-1H-indol**

327 g (2,05 mol) Isonicotinsaeurechloridhydrochlorid werden in Methylenchlorid vorgelegt (das Hydrochlorid wird durch Umsetzung von Isonicotinsaeurehydrochlorid in Thionylchlorid nach ueblichem Verfahren hergestellt). Zur Loesung tropft man bei 20 ° 532 ml (3,69 mol) Triethylamin und anschliessend 206 ml (1,84 mol) Indolin in Methylenchlorid geloest zu. Man laesst die Mischung 16 Stunden bei 20 ° ruehren. Danach wird der Ansatz mit Wasser gewaschen, die organische Phase wird getrocknet und eingedampft. Man erhaelt 2,3-Dihydro-1-(4-pyridinylcarbonyl)-1H-indol mit dem Schmelzpunkt 127 - 128 ° (Kristallisation aus Methylenchlorid/Ethanol/Hexan).

**b) 4-(2,3-Dihydro-1H-indol-1-yl-carbonyl)-1-methylpyridiniumiodid**

224 g (1 mol) 2,3-Dihydro-1-(4-pyridinylcarbonyl)-1H-indol werden in Aceton suspendiert. Man erhitzt zum Rueckfluss und tropft 137,6 ml (2,21 mol) Methyliodid zu. Nach 45 Min. erfolgt eine erneute Zugabe von 68,8 ml (1,1 mol) Methyliodid. Die Reaktionsmischung wird weitere 2 Stunden zum Rueckfluss erhitzt. Danach wird das auskrlstalllslerte Produkt abfiltriert, mit Ether gewaschen und getrocknet. Man erhaelt in Form gelber Kristalle 4-(2,3-Dihydro-1H-indol-1-yl-carbonyl)-1-methylpyridinium-iodid mit dem Schmelzpunkt 242 - 243 ° (Kristallisation Aceton/Ether).

**c) 2,3-Dihydro-1-(1,2,3,6-tetrahydro-1-methyl-4-pyridinylcarbonyl)-1H-indol**

308 g (0,84 mol) 4-(2,3-Dihydro-1H-indol-1-yl-carbonyl)-1-methylpyridinium-iodid werden in Ethanol suspendiert. Die Loesung wird auf + 10 ° gekuehlt. Unter intensivem Ruehren tropft man eine Mischung von 95,9 g (2,52 mol) Natriumborhydrid in 960 ml Wasser und 96 ml 30%ige Natronlauge ein. Anschliessend laesst man 2 Stunden bei Raumtemperatur ruehren. Danach wird die Reaktionsmischung am Rotationsverdampfer eingeengt. Der Rueckstand wird mit Eiswasser und Methylenchlorid versetzt. Die organische Phase wird abgetrennt und das Produkt daraus mit 2 n Salzsaeure extrahiert. Die salzsaure, waessrige Loesung wird mit 30%iger Natronlauge alkalisiert und das Produkt mit Methylenchlorid extrahiert. Die organische Phase wird getrocknet und eingedampft. Man erhaelt 2,3-Dihydro-1-(1,2,3,6-tetrahydro-1-methyl-4-pyridinylcarbonyl)-1H-indol mit dem Schmelzpunkt 70 - 72 ° (Kristallisation aus Ether/Petrolether).

**d) 4-(2,3-Dihydro-1H-indol-1-yl-carbonyl)-1,2,3,6-tetrahydro-1-pyridincarbaminsaeureethylester**

145,4 g (0,6 mol) 2,3-Dihydro-1-(1,2,3,6-tetrahydro-1-methyl-4-pyridinylcarbonyl)-1H-indol werden in Toluol vorgelegt. Dazu gibt man 156,6 ml N-Ethyldiisopropylamin und erwaermt auf 80 °. Bei gleicher Temperatur tropft man zur Reaktionsmischung eine Loesung von 196,8 ml (2,1 mol) Chlorameisensaeureethylester in Toluol ein. Man laesst 2 Stunden bei 80 ° ruehren und kuehlt anschliessend auf 0 ° ab. Der Ansatz wird mit Wassereis versetzt und mit 2 n Salzsaeure gewaschen. Die Toluolphase wird abgetrennt, getrocknet und eingedampft. Man erhaelt 4-(2,3-Dihydro-1H-indol-1-yl-carbonyl)-1,2,3,6-tetrahydro-1-pyridincarbaminsaeureethylester mit dem Schmelzpunkt 112 - 113 ° (Kristallisation aus Methyl-tert.butylether/Ether).

**e) cis-4,5,7a,8,9,10,11,11a-Octahydro-7-oxo-7H-indolo[1,7-bc][2,6]naphthyridin-10-carbaminsaeureethylester**

2,0 g (6,7 mmol) 4-(2,3-Dihydro-1H-indol-1-yl-carbonyl)-1,2,3,6-tetrahydro-1-pyridincarbaminsaeureethylester werden in Toluol unter Ruehren und Argon mit einer 400 Watt Quecksilber-Hochdrucklampe bestrahlt. Das Reaktionsgefaess wird mit fliessendem Wasser gekuehlt. Im Abstand von 12 Stunden wird die Tauchlampe gereinigt und die Reaktionsloesung mit 3 Mol% Aktivkohle behandelt und ueber Hyflo filtriert. Nach insgesamt 50 Stunden Bestrahlung wird das Toluol abgedampft. Das Rohprodukt wird saeulenchromatografisch (Kieselgel) gereinigt und dabei die cis/trans-Diastereomeren getrennt. Man erhaelt als Oel cis-4,5,7a,8,9,10,11,11a-Octahydro-7-oxo-7H-indolo[1,7-bc][2,6]naphthyridin-10-carbaminsaeureethylester.

IR($CH_2Cl_2$) : r = 1665 cm [e-1] (Amid C=0); 1690 cm [e-1] (Carbamat C=0).

1H-NMR(D6-DMSO), 360 MHz : d (ppm) = 1,20 (t, J = 7,5 Hz; 3H, Carbamat-CH3); 4,08 (q, J = 7,5 Hz; 2H, Carbamat-CH2).

**BEISPIEL 2: trans-4,5,7a,8,9,10,11,11a-Octahydro-7H-10-methyl-indolo[1,7-bc][2,6]naphthyridin**

Die Herstellung entspricht der fuer das cis-Diastereomer (Beispiel 1). Man erhaelt trans-4,5,7a,8,9,10,11,11a-Octahydro-7H-10-methyl-indolo[1,7-bc][2,6]naphthyridin mit dem Schmelzpunkt 102 - 103 ° (Ether/Hexan). Als Malonat mit dem Schmelzpunkt 155 156 ° (Aceton/Methanol).

Das Ausgangsmaterial kann wie folgt hergestellt werden:

**trans-4,5,7a,8,9,10,11,11a-Octahydro-7-oxo-7H-indolo[1,7-bc][2,6]naphthyridin-10-carbaminsaeureethylester**

Die Herstellung entspricht der fuer das cis-Diastereomer (Beispiel 1 e). Man erhaelt trans-4,5,7a,8,9,10,11,11a-Octahydro-7-oxo-7H-indolo[1,7-bc][2,6]naphthyridin-10-carbaminsaeureethylester mit dem Schmelzpunkt 132 - 133 ° (Kristallisation aus Methyl-tert.butylether).

Analog zu Beispiel 1 werden folgende Verbindungen der Formel I hergestellt:

| Beispiel | R | Konfig. | Smp. des Dihydrochlorids |
|---|---|---|---|
| 3 | Ethyl | (±)-cis | 280 - 281 ° |
| 4 | n-Propyl | (±)-cis | 294 - 296 ° |

**BEISPIEL 5: (+)-cis-4,5,7a,8,9,10,11,11a-Octahydro-7H-10-methyl-indolo[1,7-bc][2,6]naphthyridin**

13,57 g (59 mmol) (+/-)-cis-4,5,7a,8,9,10,11,11a-Octahydro-7H-10-methyl-indolo[1,7-bc][2,6]naphthyridin (Herstellung siehe Beispiel 1) werden in Aceton geloest. Man versetzt diese Loesung mit 23,92 g (59 mmol) (-)-Di-O,O′-p-toluyl-L-weinsaeure-monohydrat in Aceton. Die Loesung wird eingeengt, mit Ether versetzt und das auskristallisierte Salz abfiltriert und mit Aceton/Ether gewaschen. Das Salz wird bis zum konstanten Drehwert aus Ethanol/Aceton umkristallisiert [Die Mutterlaugen werden zur Gewinnung des (-)-Enantiomeren (Beispiel 6) separat gesammelt]. Man erhaelt das (-)-Di-O,O′-p-toluyl-L-tartrat mit dem Schmelzpunkt 158-159 ° und dem Drehwert - 46 ° (c = 0,5, Methanol). Aus dem Drehwert-konstanten Kristallisat wird durch Zugabe von Eis/konz. Ammoniak-Loesung und Methylenchlorid die Base freigesetzt. Man erhaelt (+)-cis-4,5,7a,8,9,10,11,11a-Octahydro-7H-10-methyl-indolo[1,7-bc][2,6]naphthyridin mit dem Drehwert + 132 ° (c = 0,5; Methanol). Die Base kristallisiert als Malonat mit dem Schmelzpunkt 129 - 130 ° (Kristallisation aus Aceton/Ether) und dem Drehwert + 71 ° (c = 0,5; Methanol).

**BEISPIEL 6: (-)-cis-4,5,7a,8,9,10,11,11a-Octahydro-7H-10-methyl-indolo[1,7-bc][2,6]naphthyridin**

Herstellung aus (+/-)-cis-4,5,7a,8,9,10,11,11a-Octahydro-7H-10-methyl-indolo[1,7-bc][2,6]naphthyridin (Beispiel 1) oder aus den gesammelten Mutterlaugen der (+)-Enantiomeren-Gewinnung unter Verwendung von (+)-Di-O,O′-p-toluyl-D-weinsaeure. Verfahren analog der Herstellung des (+)-Enantiomeren (Beispiel 5). Man erhaelt das (+)-Di-O,O′-p-toluyl-D-tartrat mit dem Schmelzpunkt 160 - 161 ° (Methanol/Aceton) und dem Drehwert + 46 ° (c = 0,5, Methanol). Nach Freisetzung der Base erhaelt man (-)-cis-4,5,7a,8,9,10,11,11a-Octahydro-7H-10-methyl-indolo[1,7-bc][2,6]naphthyridin mit dem Drehwert - 130 ° (c = 0,5, Methanol). Die Base kristallisiert als Malonat mit dem Schmelzpunkt 129-130 ° (Aceton/Ether) und dem Drehwert - 75 ° (c = 0,5, Methanol).

**BEISPIEL 7: cis-7a,8,9,10,11,11a-Hexahydro-10-methyl-7H-indolo[1,7-bc][2,6]naphthyridin**

200 mg (0,9 mmol) cis-4,5,7a,8,9,10,11,11a-Octahydro-7H-10-methyl-indolo[1,7-bc][2,6]naphthyridin (Beispiel 1) werden in Methylenchlorid geloest. Unter Ruehren addiert man bei 20 ° 2,00 g Mangan(IV)oxid (gefaellt aktiv). Man laesst 4 Stunden bei Raumtemperatur ruehren, filtriert die Reaktionsmischung anschliessend ueber Hyflo und dampft das Methylenchloridfiltrat ein. Man erhaelt nach Kristallisation aus Ether cis-7a,8,9,10,11,11a-Hexahydro-10-methyl-7H-indolo[1,7-bc][2,6]naphthyridin mit dem Schmelzpunkt 125 - 126 °. 1H-NMR, 360 MHz (CDCl3): d (ppm) = 2,31 (s; 3H, 10-N-CH3). Die Verbindung hat als Hydrogenfumarat den Schmelzpunkt 127 ° (Zers.).

**BEISPIEL 8: trans-7a,8,9,10,11,11a-Hexahydro-10-methyl-7H-indolo[1,7-bc][2,6]naphthyridin**

Die Herstellung entspricht der des cis-Diastereomeren (Beispiel 7). Man erhaelt trans-7a,8,9,10,11,11a-Hexahydro-10-methyl-7H-indolo[1,7-bc][2,6]naphthyridin mit dem Schmelzpunkt 143 - 145 ° (Kristallisation aus Ethanol). 1H-NMR, 360 MHz(CDCl3): d (ppm) = 2,45 (s; 3H, 10-N-CH3); 6,44 (m; 1H, C-4-H).

Das Ausgangsmaterial kann wie folgt hergestellt werden:

**a) trans-4,5,7a,8,9,10,11,11a-Octahydro-7-oxo-7H-indolo[1,7-bc][2,6]naphthyridin-10-carbaminsaeureethylester**

Die Herstellung entspricht der fuer das cis-Diastereomer (Beispiel 1 e). Man erhaelt trans-4,5,7a,8,9,10,11,11a-Octahydro-7-oxo-7H-indolo[1,7-bc][2,6]naphthyridin-10-carbaminsaeureethylester mit dem Schmelzpunkt 132 - 133 ° (Kristallisation aus Methyl-tert.butylether).

**b) trans-4,5,7a,8,9,10,11,11a-Octahydro-7H-10-methyl-indolo[1,7-bc][2,6]naphthyridin**

Die Herstellung entspricht der fuer das cis-Diastereomer (Beispiel 1). Man erhaelt trans-4,5,7a,8,9,10,11,11a-Octahydro-7H-10-methyl-indolo[1,7-bc][2,6]naphthyridin mit dem Schmelzpunkt 102 - 103 ° (Ether/Hexan). Als Malonat mit dem Schmelzpunkt 155 - 156 ° (Aceton/Methanol).

Analog zu Beispiel 7 werden auch folgende Verbindungen der Formel I hergestellt, worin $R_2$, $R_4$, $R_5$, X und Y Wasserstoff bedeuten:

| Beispiel | $R_1$ | $R_3$ | Konfig. | Smp. |
|---|---|---|---|---|
| 9 | Ethyl | H | (±)-cis | 156 - 158 ° (Hydrogenmaleinat) |
| 10 | n-Propyl | H | (±)-cis | 105 - 107 ° (Hydrogenmaleinat) |
| 11 | Methyl | 2-Cl | (±)-trans | 324 ° (Zers.) (Hydrochlorid) |

**BEISPIEL 12: (-)-cis-7a,8,9,10,11,11a-Hexahydro-10-methyl-7H-indolo[1,7-bc][2,6]naphthyridin**

16,10 g (71 mmol) (+/-)-cis-7a,8,9,10,11,11a-Hexahydro-10-methyl-7H-indolo[1,7-bc][2,6]naphthyridin (Herstellung siehe Beispiel 7) werden in Aceton geloest. Man versetzt diese Loesung mit 28,80 g (71 mmol) (-)-Di-O,O′-p-toluyl-L-weinsaeure-monohydrat (in Aceton). Die Loesung wird eingeengt, mit Ether versetzt und das auskristallisierte Salz abfiltriert und mit Aceton/Methanol gewaschen. Das Salz wird bis zum konstanten Drehwert umkristallisiert aus Methylenchlorid/Methanol. (Die Mutterlaugen werden separat gesammelt, vgl. Beispiel 13). Aus dem Drehwert-konstanten Kristallisat wird durch Zugabe von Eis/konz. Ammoniak-Loesung und Methylenchlorid die Base freigesetzt.

Man erhaelt (-)-cis-7a,8,9,10,11,11a-Hexahydro-10-methyl-7H-indolo[1,7-bc][2,6]naphthyridin vom Schmelzpunkt 99 - 100 ° und dem Drehwert $[\alpha]_D^{20}$ = - 50 ° (c = 0,25, $CH_2Cl_2$).

**BEISPIEL 13: (+)-cis-7a,8,9,10,11,11a-Hexahydro-10-methyl-7H-indolo[1,7-bc][2,6]naphthyridin**

Herstellung aus (+/-)-cis-7a,8,9,10,11,11a-Hexahydro-10-methyl-7H-indolo[1,7-bc][2,6]naphthyridin (Beispiel 7) oder aus den gesammelten Mutterlaugen der (-)-Enantiomer-Gewinnung unter Verwendung von (+)-Di-O,O′-p-toluyl-D-weinsaeure. Verfahren analog der Herstellung des (-)-Enantiomeren (Beispiel 12). Man erhaelt nach Freisetzung der Base (+)-cis-7a,8,9,10,11,11a-Hexahydro-10-methyl-7H-indolo[1,7-bc][2,6]naphthyridin vom Schmelzpunkt 101 - 102 ° (Ether/Hexan) mit dem Drehwert $[\alpha]_D^{20}$ = + 49 ° (c = 0,25, $CH_2Cl_2$).

## Patentansprüche

1. Ein 4,5,7a,8,9,10,11,11a-Octahydro-7H-indolo[1,7-bc][2,6]-naphthyridin in Form der freien Base oder eines physiologisch verträglichen Säureadditionssalzes.

2. Eine Verbindung der Formel I,

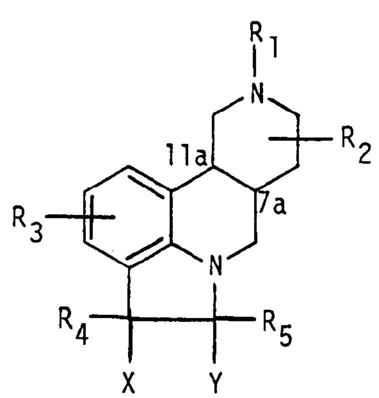

I

worin

$R_1$ Wasserstoff, Alkyl, Alkylcarbonylalkyl, Arylcarbonylalkyl, Aralkyl oder gegebenenfalls durch Alkyl oder Aryl mono- oder disubstituiertes Carbamoylalkyl bedeutet,

$R_2$ für eine der unter $R_1$ angegebenen Bedeutungen steht und zusätzlich Trifluormethyl, Alkoxy oder Alkylthio bedeuten kann,

$R_3$, $R_4$ und $R_5$ unabhängig voneinander je Wasserstoff, Halogen, Alkyl, Alkoxy, Alkylthio oder Trifluormethyl bedeuten und

X und Y je für Wasserstoff stehen oder zusammen eine einfache Bindung bilden,

in Form der freien Base oder eines Säureadditionssalzes.

**3.** Eine Verbindung gemäss Anspruch 2, worin $R_1$ Alkyl mit 1 bis 4 Kohlenstoffatomen bedeutet und $R_2$, $R_3$, $R_4$, $R_5$, X und Y je Wasserstoff bedeuten.

**4.** Eine Verbindung gemäss Anspruch 2, worin $R_1$ Alkyl mit 1 bis 4 Kohlenstoffatomen bedeutet, $R_2$, $R_3$, $R_4$ und $R_5$ je Wasserstoff bedeuten und X und Y zusammen eine einfache Bindung bilden.

**5.** Das (+)-cis-4,5,7a,8,9,10,11,11a-Octahydro-7H-10-methyl-indolo[1,7-bc][2,6]naphthyridin in Form der freien Base oder eines Säureadditionssalzes.

**6.** Verfahren zur Herstellung einer Verbindung gemäss Anspruch 2, dadurch gekennzeichnet, dass man

a) zur Herstellung der Verbindungen der Formel Ia,

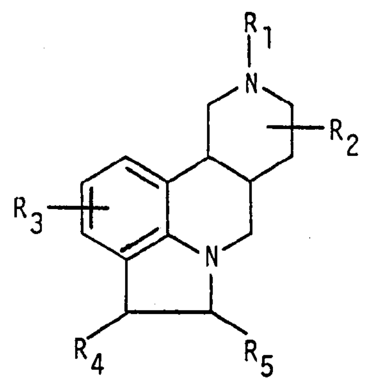

Ia

worin $R_1$, $R_2$, $R_3$, $R_4$ und $R_5$ wie im Anspruch 2 definiert sind, in Verbindungen der Formel II,

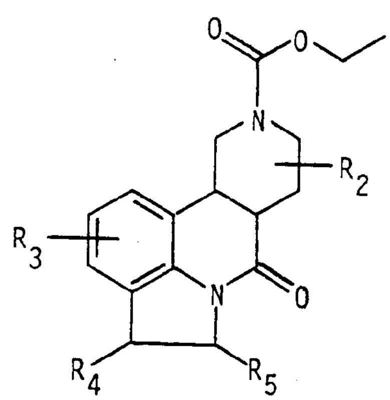

II

worin $R_2$, $R_3$, $R_4$ und $R_5$ wie im Anspruch 2 definiert sind, die 100-Ethoxycarbonylgruppe unter Reduktion der 7-Oxogruppe durch die Gruppe $R_1$ ersetzt, oder

b) zur Herstellung der Verbindungen der Formel Ib,

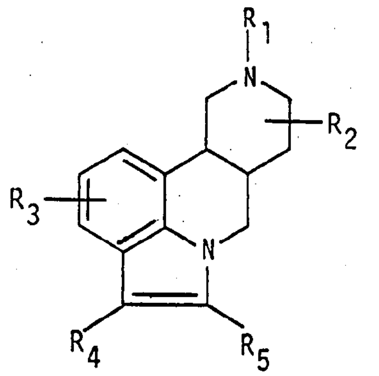

Ib

worin $R_1$, $R_2$, $R_3$, $R_4$ und $R_5$ wie im Anspruch 2 definiert sind, Verbindungen der Formel Ia oxidiert, und die erhaltenen Verbindungen der Formel I in Form der freien Basen oder in Säureadditionssalzform gewinnt.

7. Eine Verbindung gemäss einem der Ansprüche 1 bis 5, in freier oder physiologisch verträglicher Säureadditionssalzform, zur Anwendung als Pharmazeutikum.

8. Eine Verbindung gemäss einem der Ansprüche 1 bis 5, in freier oder physiologisch verträglicher Säureadditionssalzform, zur prophylaktischen Behandlung der Migräne oder zur Behandlung von Störungen wie Angst, Depression, Schizophrenie, Autismus, Panikattacken, Zwangskrankheiten, Priapismus, Bulimie und Zuständen mit erhöhtem intracranialem Druck.

9. Eine pharmazeutische Zusammensetzung, enthaltend eine Verbindung gemäss einem der Ansprüche 1 bis 5, in freier oder physiologisch verträglicher Säureadditionssalzform.

10. Die Anwendung einer Verbindung gemäss einem der Ansprüche 1 bis 5, für die Herstellung einer pharmazeutischen Zusammensetzung zur prophylaktischen Behandlung der Migräne oder zur Behandlung von Störungen wie Angst, Depression, Schizophrenie, Autismus, Panikattacken, Zwangskrankheiten, Priapismus, Bulimie und Zuständen mit erhöhtem intracranialem Druck.

**Patentansprüche für folgende Vertragsstaaten : ES, GR**

1. Verfahren zur Herstellung einer Verbindung der Formel 1,

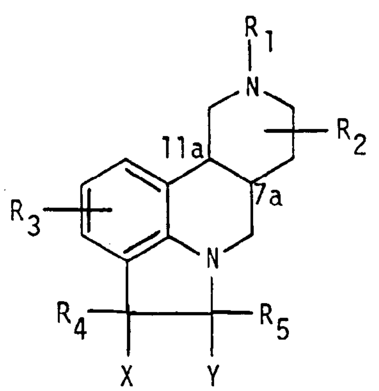

I

worin
$R_1$ Wasserstoff, Alkyl, Alkylcarbonylalkyl, Arylcarbonylalkyl, Aralkyl oder gegebenenfalls durch Alkyl oder Aryl mono- oder disubstituiertes Carbamoylalkyl bedeutet,
$R_2$ für eine der unter $R_1$ angegebenen Bedeutungen steht und zusätzlich Trifluormethyl, Alkoxy oder Alkylthio bedeuten kann,

$R_3$, $R_4$ und $R_5$ unabhängig voneinander je Wasserstoff, Halogen, Alkyl, Alkoxy, Alkylthio oder Trifluormethyl bedeuten und
X und Y je für Wasserstoff stehen oder zusammen eine einfache Bindung bilden,
in Form der freien Base oder eines Säureadditionssalzes, dadurch gekennzeichnet, dass man

a) zur Herstellung der Verbindungen der Formel Ia,

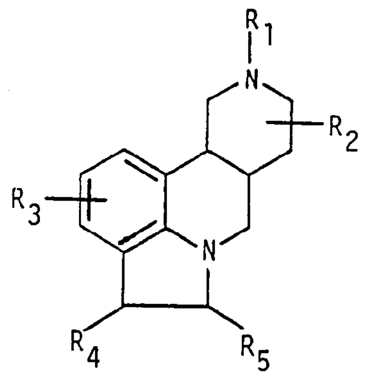

Ia

worin $R_1$, $R_2$, $R_3$, $R_4$ und $R_5$ obige Bedeutung besitzen, in Verbindungen der Formel II,

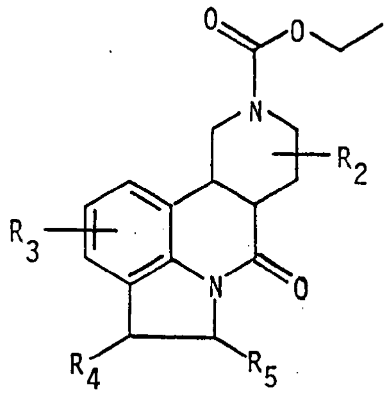

II

worin $R_2$, $R_3$, $R_4$ und $R_5$ obige Bedeutung besitzen, die 10-Ethoxycarbonylgruppe unter Reduktion der 7-Oxogruppe durch die Gruppe $R_1$ ersetzt, oder

b) zur Herstellung der Verbindungen der Formel Ib,

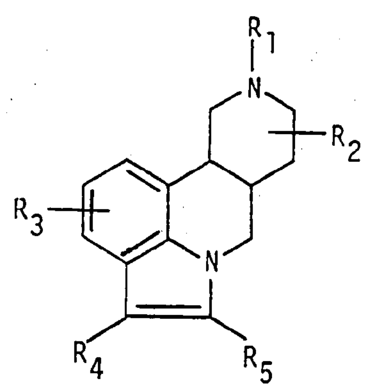

Ib

worin $R_1$, $R_2$, $R_3$, $R_4$ und $R_5$ obige Bedeutung besitzen, Verbindungen der Formel Ia oxidiert, und die erhaltenen Verbindungen der Formel I in Form der freien Basen oder in Säureadditionssalzform gewinnt.

**2.** Verfahren gemäss Anspruch 1, dadurch gekennzeichnet, dass man Verbindungen der Formel I herstellt, worin $R_1$ Alkyl mit 1 bis 4 Kohlenstoffatomen bedeutet und $R_2$, $R_3$, $R_4$, $R_5$, X und Y je Wasserstoff bedeuten.

**3.** Verfahren gemäss Anspruch 1, dadurch gekennzeichnet, dass man Verbindungen der Formel I herstellt, worin $R_1$ Alkyl mit 1 bis 4 Kohlenstoffatomen bedeutet, $R_2$, $R_3$, $R_4$ und $R_5$ je Wasserstoff bedeuten und X und Y zusammen eine einfache Bindung bilden.

**4.** Verfahren gemäss Anspruch 1, zur Herstellung des (+)-cis-4,5,7a,8,9,10,11,11a-Octahydro-7H-10-methyl-indolo[1,7-bc][2,6]naphthyridin in Form der freien Base oder eines Säureadditionssalzes.

**5.** Verfahren zur Herstellung einer pharmazeutischen Zusammensetzung, enthaltend eine Verbindung der Formel I in Form der freien Base oder eines physiologisch verträglichen Säureadditionssalzes, dadurch gekennzeichnet, dass man die Verbindung der Formel I mit einem pharmazeutischen Hilfs- oder Trägerstoff vermischt.

Europäisches Patentamt

# EUROPÄISCHER RECHERCHENBERICHT

Nummer der Anmeldung: EP 91810667.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
|---|---|---|---|
| A | CHEMICAL ABSTRACTS, Band 110, Nr. 13, 27. März 1989, Columbus, Ohio, USA C. PAPAGEORGIOU et al. "Acid-catalyzed rearrangements for a diastereoselective entry into a new fused hexacyclic heterocycle: (5RS,7aRS,12RS,14aRS)-4,5,7,7a,11,12,14,14a-octahydro-5,12-dimethyldiindolo(1,7-bc:1',7'-gh)(2,6)naphthyridine" Seite 681, Spalte 2, Zusammenfassung-Nr. 114 711c & Helv. Chim. Acta 1988, 71(5) 1079-83 ---- | 1,2 | C 07 D 471/16<br>A 61 K 31/47<br>//(C 07 D 471/16<br>C 07 D 221:00<br>C 07 D 221/00<br>C 07 D 209:00) |

RECHERCHIERTE SACHGEBIETE (Int. Cl.⁵)

C 07 D 471/00

Der vorliegende Recherchenbericht wurde für alle Patentansprüche erstellt.

| Recherchenort | Abschlußdatum der Recherche | Prüfer |
|---|---|---|
| WIEN | 22-10-1991 | ONDER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82